# EUROPEAN PATENT APPLICATION

(11) **EP 3 885 341 A1**
(43) Date of publication of application: **29.09.2021**
(21) Application number: 18944314.6
(22) Date of filing: 29.12.2018
(51) Int. Cl.: C07D 249/08, C07D 401/06

(54) **PREPARATION METHOD FOR EFINACONAZOLE**

(71) Applicant: Viwit Pharmaceutical Co., Ltd., Tengzhou, Shandong 277500 (CN)
(72) Inventor: WEI, Yanjun, Shandong 277500 (CN); WANG, Jian, Shandong 277500 (CN); XING, Yanping, Shandong 277500 (CN)
(74) Representative: Engelhard, Markus
(86) International application number: PCT/CN2018/125609
(87) International publication number: WO 2020/133411

(57) **Abstract**

The present invention provides a preparation method for Efinaconazole, comprising the following steps: in the presence of a bromide and a base, subjecting (2R,3S)-2-(2,4-difluorophenyl)-3-methyl-2-[(1H-1,2,4-triazole-1-yl)methyl]oxirane and an inorganic acid salt of 4-methylenepiperidine to a ring-opening addition reaction in a reaction solvent, to obtain Efinaconazole. The preparation method for Efinaconazole in the present invention has mild reaction conditions, high product yield, high purity, and low production costs, and is suitable for industrial production.

## Description

### TECHNICAL FIELD

The present invention relates to the field of drug synthesis, in particular to a preparation method for Efinaconazole or its salt.

### BACKGROUND

Efinaconazole (generic name: Efinaconazole, trade name: Jublia) is a topical triazole antifungal drug, which is used to treat tinea unguium with good therapeutic effect and low side effects. Efinaconazole was developed by Dow Pharmaceutical and was approved by the FDA on June 6, 2014 as a topical triazole antifungal drug. It is clinically used to treat Onychomycosis with a 10% solution. Efinaconazole has a chemical name of (2R,3R)-2-(2,4-difluorophenyl)-3-(4-methylenepiperidin-1-yl)-1-(1H-1,2,4-triazol-1-yl)butan-2-o 1, CAS registration number of 164650-44-6, and structural formula shown in formula I:

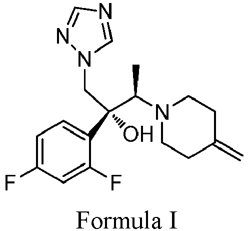

Patent CN1122598B discloses a method for preparing Efinaconazole using (2R,3S)-2-(2,4-difluorophenyl)-3-methyl-2-[(1H-1,2,4-triazol-1-yl)methyl]oxirane and 4-methylenepiperidine hydrochloride. In this method, a large excess of 4-methylenepiperidine hydrochloride is usually used, and the reaction requires a long time and high temperature heating. The method has the disadvantages of high cost, low yield, producing many by-products and low product purity.

Patent CN103080100B discloses a preparation method for Efinaconazole, which adopts basically the same route as patent CN1122598B, but uses different reaction conditions. The change of reaction conditions has improved the reaction yield to a certain extent, wherein the use of hydrobromide or hydroiodide of 4-methylenepiperidine has obtained a higher yield. However, the yield is still low when using 4-methylenepiperidine hydrochloride. The method is prone to produce more impurities, which ultimately affects the purity and yield of Efinaconazole.

Keiji Tamura et al. reports in a non-patent literature (The Journal of Organic Chemistry, 2014, 79(7), 3272-3278) a microwave heating method using ethanol as a solvent, to prepare Efinaconazole by reacting (2R,3S)-2-(2,4-difluorophenyl)-3-methyl-2-[(1H-1,2,4-triazol-1-yl) methyl]oxirane and 4-methylenepiperidine, but the reaction requires high temperature heating.

Therefore, in this field, there is still a need to further develop preparation methods for Efinaconazole with mild reaction conditions, high yield, high purity, and being suitable for industrial production.

### SUMMARY

In view of the problems in the preparation of Efinaconazole in the prior art, such as incomplete reaction of raw materials, low yield, low purity, and high cost, the present invention provides a preparation method for Efinaconazole. The method of the present invention has the advantages of mild reaction conditions, high product yield, high purity, low production cost, and being suitable for industrial production.

The preparation method for Efinaconazole of the present invention comprises the following steps:
in the presence of a bromide and a base, subjecting (2R,3S)-2-(2,4-difluorophenyl) -3-methyl-2-[(1H-1,2,4-triazol-1-yl)methyl]oxirane (the compound of formula A) and an acid addition salt of 4-methylenepiperidine (the compound of formula B) to a ring-opening addition reaction in a reaction solvent to obtain Efinaconazole (the compound of formula I) 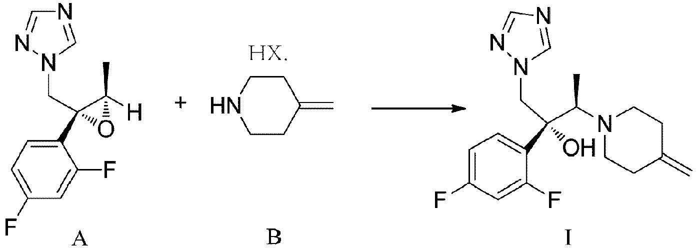 wherein, the X in the compound of formula B is Cl, Br or I, preferably Cl.

According to the preparation method for Efinaconazole of the present invention, the base used in the reaction is one or more selected from potassium hydroxide, sodium hydroxide, potassium carbonate and lithium carbonate, preferably potassium hydroxide.

According to the preparation method for Efinaconazole of the present invention, the reaction solvent used in the reaction is selected from acetonitrile, DMF, DME or DMSO, preferably acetonitrile.

According to the preparation method for Efinaconazole of the present invention, the bromide used in the reaction is lithium bromide or magnesium bromide, preferably lithium bromide.

According to the preparation method for Efinaconazole of the present invention, the molar ratio of the bromide to the compound of formula A is 1.5-2.5:1, preferably 2.0:1.

According to the preparation method for Efinaconazole of the present invention, the mass ratio of the reaction solvent to the compound of formula A is 2-5:1, preferably 2:1.

According to the preparation method for Efinaconazole of the present invention, the molar ratio of the base to the compound of formula A is 1.0-1.8:1, preferably 1.3:1.

According to the preparation method for Efinaconazole of the present invention, the molar ratio of the compound of formula A to the compound of formula B is 1:1.0-1:1.8, preferably 1:1.3.

According to the preparation method for Efinaconazole of the present invention, the reaction can be carried out under heating conditions, the reaction time varies according to the reaction temperature and the solvent used, and the reaction is carried out under atmospheric pressure. Specifically, the temperature of the ring-opening addition reaction is 70-90°C, preferably 85°C; the time of the ring-opening addition reaction is 14-24h, preferably 20h.

According to the preparation method for Efinaconazole of the present invention, the method further comprises: after completion of the reaction, subjecting a post-treatment to the product to obtain a crude Efinaconazole, wherein the post-treatment does not comprise a crystallization step.

According to the preparation method for Efinaconazole of the present invention, the post-treatment is adding ethanol and water to the product, wherein the mass ratio of ethanol to water is 5:6-6:6, preferably 5.5:6.

According to the preparation method for Efinaconazole of the present invention, the method further comprises a crystallization step.

According to the preparation method for Efinaconazole of the present invention, the crystallization step comprises: adding the crude Efinaconazole to the crystallization solvent, stirring and crystallizing, and filtering to obtain pure Efinaconazole.

According to the preparation method for Efinaconazole of the present invention, in the crystallization step, the crystallization temperature is 0-25°C, preferably 0-10°C, more preferably 5-8°C.

According to the preparation method for Efinaconazole of the present invention, in the crystallization step, the time for stirring and crystallizing is 2-15 hours, preferably 4-14 hours.

According to the preparation method for Efinaconazole of the present invention, the crystallization solvent used in the crystallization step can be selected from n-heptane, isopropyl ether, acetonitrile/water, acetone/water, ethanol/water, ethanol/acetonitrile/water, diethyl ether /n-hexane or cyclohexane, preferably cyclohexane.

According to the preparation method for Efinaconazole of the present invention, the obtained Efinaconazole can form a salt with an acid. The obtained Efinaconazole can form methanesulfonate with methanesulfonic acid, and form p-toluenesulfonate with p-toluenesulfonic acid.

### DESCRIPTION OF THE DRAWINGS

Figure 1 is a ¹H-NMR spectrum of the pure Efinaconazole obtained in Example 2 of the present application.
Figure 2 is a ¹³C-NMR spectrum of the pure Efinaconazole obtained in Example 2 of the present application.

### EMBODIMENT

The following examples are used to further explain the present invention, but they do not constitute a limitation or definition to the scope of the present invention.

(2R,3S)-2-(2,4-difluorophenyl)-3-methyl-2-[(1H-1,2,4-triazol-1-yl)methyl]oxirane used in the following examples was prepared by the method of Example 2 in patent application CN106608867A; 4-methylenepiperidine hydrochloride can be prepared by reacting 4-methylenepiperidine with hydrochloric acid, for example, 4-methylenepiperidine hydrochloride can be prepared according to the following reaction.

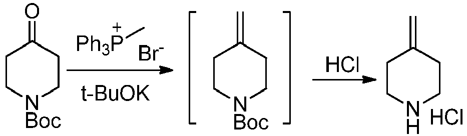

### Example 1 Preparation of crude Efinaconazole

Into a 2L reactor, 300g of acetonitrile, 4-methylenepiperidine hydrochloride (103.6g, 0.78mol) and NaOH (31.0g, 0.78mol) were added, the mixture was stirred at 25-35 □ for 1h, LiBr (103.6g, 1.19mol) and (2R,3S)-2-(2,4-difluorophenyl)-3-methyl-[(1H-1,2,4-triazol-1-yl) methyl]oxirane (150.0g, 0.60mol) were added. The heating was started and the temperature was raised until reflux, the temperature was maintained at around 85-90 □ and the mixture was stirred for 20h, then the heating was stopped. After filtration, the filtrate was concentrated under reduced pressure until no fraction appeared (oily). Ethanol (825g) was added, filtered, the filtrate was cooled to 0-10□, and 900g of purified water was added dropwise. After completion of the dropwise addition, the mixture was stirred for 2 hours with the temperature maintained, filtered with suction, and the filter cake was vacuum dried at 47□ to obtain 167.0g of crude Efinaconazole as an off-white solid with a yield of 80.27%. The purity was 99.25%.

### Example 2 Preparation of pure Efinaconazole

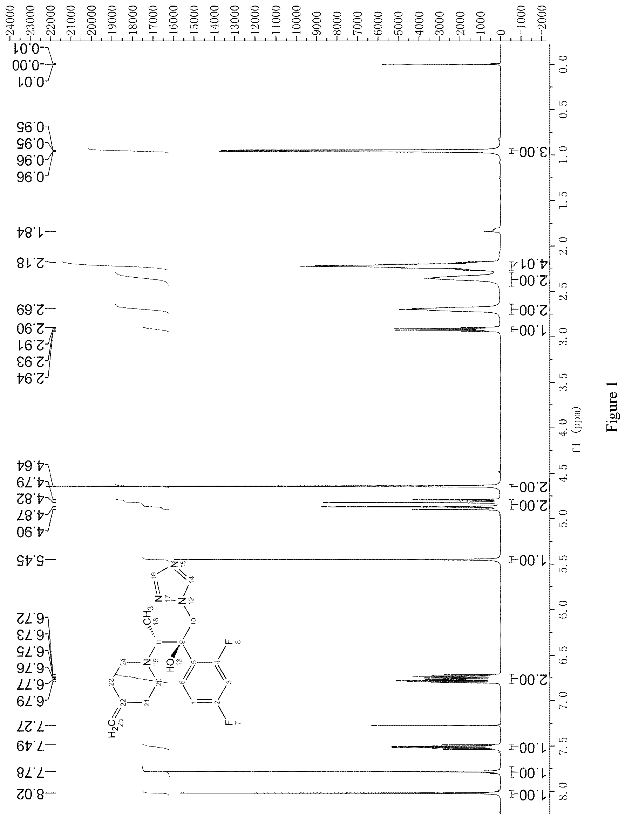

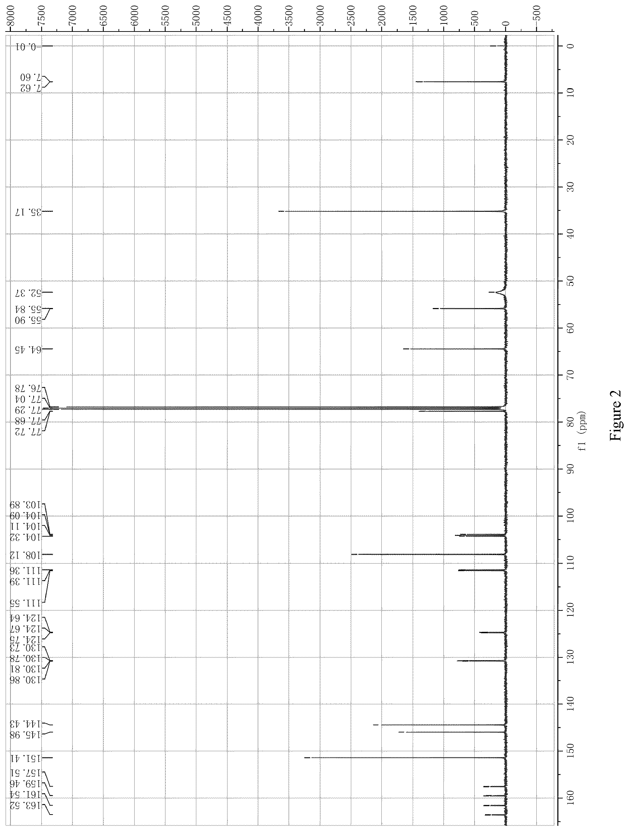

450g of absolute ethanol was added into 150g of crude Efinaconazole and the crude Efinaconazole was dissolved until clear, the solution was stirred at room temperature for 40min, filtered, and the filtrate was cooled to 0-10°C, and 900g of purified water was added dropwise. After completion of the dropwise addition, the mixture was stirred for 15h with the temperature maintained, filtered with suction, the filter cake was washed with 450g ethanol/water=1/2 and vacuum dried at 47□ to obtain 144g of pure Efinaconazole as a white solid with a yield of 96.0% and a purity of 99.88%. The ¹H-NMR spectrum and ¹³C-NMR spectrum of the obtained pure Efinaconazole are shown in Figure 1 and Figure 2 respectively.

### Example 3 Preparation of crude Efinaconazole

Into a 2L reactor, 400g of acetonitrile, 4-methylenepiperidine hydrochloride (138.2g, 1.03mol) and KOH (57.7g, 1.03mol) were added, then LiBr (138.2g, 1.59mol) and (2R,3S)-2-(2,4-difluorophenyl)-3-methyl-[(1H-1,2,4-triazol-1-yl)methyl]oxirane (200.0g, 0.80mol) were added. The heating was started and the temperature was raised until reflux, the temperature was maintained at around 85-90□ and the mixture was stirred for 20h, then the heating was stopped. After filtration, the filtrate was concentrated under reduced pressure until no fraction appeared (oily). Ethanol (1.1Kg) was added, filtered, the filtrate was cooled to 0-10□, and 1.2Kg of purified water was added dropwise. After completion of the dropwise addition, the mixture was stirred for 2h with the temperature maintained, filtered with suction, and the filter cake was vacuum dried at 47□ to obtain 230.48g of crude Efinaconazole as an off-white solid with a yield of 83.1%. The purity was 99.01%.

### Example 4 Preparation of pure Efinaconazole

450g of absolute ethanol was added into 180g of crude Efinaconazole and the crude Efinaconazole was dissolved until clear, the solution was stirred at room temperature for 20min, filtered, washed with 90g of ethanol, the filtrate was cooled to 0-10°C, and 1.1Kg of purified water was added dropwise. After completion of the dropwise addition, the mixture was stirred for 14h with the temperature maintained, filtered with suction, the filter cake was washed with 540g ethanol/water=1/2 and vacuum dried at 47□ to obtain 169.0g of Efinaconazole as a white solid with a yield of 93.89% and a purity of 99.73%. The ¹H-NMR spectrum and ¹³C-NMR spectrum of the obtained pure Efinaconazole are the same as Figure 1 and Figure 2 respectively.

### Example 5 Preparation of crude Efinaconazole

Into a 20L reactor, 4.0Kg of acetonitrile, 4-methylenepiperidine hydrochloride (1.38Kg, 10.3mol) and KOH (577g, 10.3mol) were added, then LiBr (1.38Kg, 15.9mol) and (2R,3S)-2-(2,4-difluorophenyl)-3-methyl-[(1H-1,2,4-triazol-1-yl)methyl]oxirane (2.0Kg, 7.96mol) were added. The heating was started and the temperature was raised until reflux, the temperature was maintained at around 85-90□ and the mixture was stirred for 20h, the heating was stopped, filtered, the filtrate was concentrated under reduced pressure until no fraction appeared (oily). Ethanol (11Kg) was added, filtered, the filtrate was cooled to 0-10□ under stirring, and 12Kg of purified water was added dropwise. After completion of the dropwise addition, the mixture was stirred for 6h with the temperature maintained, filtered with suction, and the filter cake was vacuum dried at 47□ to obtain 2.41Kg of crude Efinaconazole as an off-white solid with a yield of 86.91%. The purity was 99.73%.

### Example 6 Preparation of pure Efinaconazole

6.6Kg of absolute ethanol was added into 2.2Kg of crude Efinaconazole and the crude Efinaconazole was dissolved until clear under stirring, the solution was stirred at room temperature for 40min, cooled to 0-10°C, and 13.2Kg of purified water was added dropwise. After completion of the dropwise addition, the mixture was stirred for 15h with the temperature maintained, filtered with suction, the filter cake was washed with 6.6Kg ethanol/water=1/2, and vacuum dried at 47□ after suction to obtain 1.95Kg of Efinaconazole as a white solid with a yield of 88.64% and a purity of 99.84%. The ¹H-NMR spectrum and ¹³C-NMR spectrum of the obtained pure Efinaconazole are the same as Figure 1 and Figure 2 respectively.

### Example 7 Preparation of crude Efinaconazole

Into a 100L reactor, 15.5Kg of acetonitrile, 4-methylenepiperidine hydrochloride (5.5Kg, 41.5mol) and KOH (2.3Kg, 41.5mol) were added, then LiBr (5.5Kg, 63.8mol) and (2R,3S)-2-(2,4-difluorophenyl)-3-methyl-[(1H-1,2,4-triazol-1-yl)methyl]oxirane (8.0Kg, 31.8mol) were added. The heating was started and the temperature was raised until reflux, the temperature was maintained at around 85-90□ and the mixture was stirred for 20h, the heating was stopped, cooled, filtered, the filtrate was concentrated under reduced pressure until no fraction appeared (oily). Ethanol (44Kg) was added, filtered, the filtrate was cooled to 0-10□ under stirring, and 48.0Kg of purified water was added dropwise. After completion of the dropwise addition, the mixture was stirred for 6h with the temperature maintained, centrifuged for 2h, and the filter cake was vacuum dried at 47□ to obtain 8.55Kg of crude Efinaconazole as an off-white solid with a yield of 77.05%. The purity was 99.38%.

### Example 8 Preparation of pure Efinaconazole

20.0Kg of absolute ethanol was added into 8.0Kg of crude Efinaconazole and the crude Efinaconazole was dissolved until clear under stirring, the solution was stirred at room temperature for 30min, the suction filtration was started with 800g activated carbon lined in the suction filter barrel, washed with 4.0Kg ethanol, the filtrate was cooled to 0-10°C, and 48.0Kg of purified water was added dropwise. After completion of the dropwise addition, the mixture was stirred for 14h with the temperature maintained, centrifuged, the product was washed with 24.0Kg ethanol/water=1/2, centrifuged again for 2h, and then vacuum dried at 47□ to obtain 7.51Kg of Efinaconazole as a white solid with a yield of 93.88% and a purity of 99.84%. The ¹H-NMR spectrum and ¹³C-NMR spectrum of the obtained pure Efinaconazole are the same as Figure 1 and Figure 2 respectively.

In the post-treatment for preparing crude Efinaconazole (Examples 1, 3, 5 and 7), the mass ratio of ethanol to water added is 5:6-6:6, preferably 5.5:6. In the following, further examples are given where the mass ratio of ethanol to water is 4:6-6:6 in the post-treatment for preparing crude Efinaconazole.

### Example 9

Into a reaction flask, 20g of acetonitrile, 4-methylenepiperidine hydrochloride (8.0g, 59.71mmol) and NaOH (2.4g, 59.7mmol) were added, then LiBr (6.9g, 95.5mmol) and (2R,3S)-2-(2,4-difluorophenyl)-3-methyl-[(1H-1,2,4-triazol-1-yl)methyl]oxirane (10g, 39.80 mmol) were added. The heating was started and the temperature was raised to around 85-90□, and the mixture was stirred for 20h, the heating was stopped, cooled, filtered, and the filtrate was concentrated under reduced pressure until no fraction appeared. 40g of ethanol was added, and 60g of purified water was added dropwise. The solid was separated out under stirring, filtered, and the filter cake was vacuum dried at 47□ to obtain 12.36g of crude Efinaconazole as an off-white solid with a yield of 89.11% and a HPLC purity of 97.76%.

### Example 10

Into a reaction flask, 20g of acetonitrile, 4-methylenepiperidine hydrochloride (8.0g, 59.71mmol) and NaOH (2.4g, 59.7mmol) were added, then LiBr (6.9g, 95.5mmol) and (2R,3S)-2-(2,4-difluorophenyl)-3-methyl-[(1H-1,2,4-triazol-1-yl)methyl]oxirane (10g, 39.80 mmol) were added. The heating was started and the temperature was raised to around 85-90□, and the mixture was stirred for 20h, the heating was stopped, cooled, filtered, and the filtrate was concentrated under reduced pressure until no fraction appeared. 45g of ethanol was added, and 60g of purified water was added dropwise. The solid was separated out under stirring, filtered, and the filter cake was vacuum dried at 47□ to obtain 12.61g of crude Efinaconazole as an off-white solid with a yield of 90.91% and a HPLC purity of 95.73%.

### Example 11

Into a reaction flask, 20g of acetonitrile, 4-methylenepiperidine hydrochloride (8.0g, 59.71mmol) and NaOH (2.4g, 59.7mmol) were added, then LiBr (6.9g, 95.5mmol) and (2R,3S)-2-(2,4-difluorophenyl)-3-methyl-[(1H-1,2,4-triazol-1-yl)methyl]oxirane (10g, 39.80 mmol) were added. The heating was started and the temperature was raised to around 85-90□, and the mixture was stirred for 20h, the heating was stopped, cooled, filtered, and the filtrate was concentrated under reduced pressure until no fraction appeared. 50g of ethanol was added, and 60g of purified water was added dropwise. The solid was separated out under stirring, filtered, and the filter cake was vacuum dried at 47□ to obtain 12.52g of crude Efinaconazole as an off-white solid with a yield of 90.27% and a HPLC purity of 97.27%.

### Example 12

Into a reaction flask, 20g of acetonitrile, 4-methylenepiperidine hydrochloride (8.0g, 59.71mmol) and NaOH (2.4g, 59.7mmol) were added, then LiBr (6.9g, 95.5mmol) and (2R,3S)-2-(2,4-difluorophenyl)-3-methyl-[(1H-1,2,4-triazol-1-yl)methyl]oxirane (10g, 39.80 mmol) were added. The heating was started and the temperature was raised to around 85-90□, and the mixture was stirred for 20h, the heating was stopped, cooled, filtered, and the filtrate was concentrated under reduced pressure until no fraction appeared. 80g of ethanol was added, and 90g of purified water was added dropwise. The solid was separated out under stirring, filtered, and the filter cake was vacuum dried at 47□ to obtain 17.44g of crude Efinaconazole as an off-white solid with a yield of 83.82% and a HPLC purity of 99.05%.

### Example 13

Into a reaction flask, 30g of acetonitrile, 4-methylenepiperidine hydrochloride (12.0g, 89.56mmol) and NaOH (3.6g, 89.56mmol) were added, then LiBr (13.8g, 95.5mmol) and (2R,3S)-2-(2,4-difluorophenyl)-3-methyl-[(1H-1,2,4-triazol-1-yl)methyl]oxirane (15g, 59.71 mmol) were added. The heating was started and the temperature was raised to around 85-90□, and the mixture was stirred for 20h, the heating was stopped, cooled, filtered, and the filtrate was concentrated under reduced pressure until no fraction appeared. 90g of ethanol was added, and 90g of purified water was added dropwise. The solid was separated out under stirring, filtered, and the filter cake was vacuum dried at 47□ to obtain 17.88g of crude Efinaconazole as an off-white solid with a yield of 85.94% and a HPLC purity of 99.53%.

### Example 14

Into a reaction flask, 75g of acetonitrile, 4-methylenepiperidine hydrochloride (12.0g, 89.56mmol) and NaOH (3.6g, 89.56mmol) were added, then LiBr (13.8g, 95.5mmol) and (2R,3S)-2-(2,4-difluorophenyl)-3-methyl-[(1H-1,2,4-triazol-1-yl)methyl]oxirane (15g, 59.71 mmol) were added. The heating was started and the temperature was raised to around 85-90□, and the mixture was stirred for 20h, the heating was stopped, cooled, filtered, and the filtrate was concentrated under reduced pressure until no fraction appeared. 90g of ethanol was added, and 90g of purified water was added dropwise. The solid was separated out under stirring, filtered, and the filter cake was vacuum dried at 47□ to obtain 14.82g of crude Efinaconazole as an off-white solid with a yield of 71.22% and a HPLC purity of 99.45%.

### Example 15

Into a reaction flask, 30g of acetonitrile, 4-methylenepiperidine hydrochloride (12.0g, 89.56mmol) and NaOH (3.6g, 89.56mmol) were added, then LiBr (13.8g, 95.5mmol) and (2R,3S)-2-(2,4-difluorophenyl)-3-methyl-[(1H-1,2,4-triazol-1-yl)methyl]oxirane (15g, 59.71 mmol) were added. The heating was started and the temperature was raised to around 85-90□, and the mixture was stirred for 24h, the heating was stopped, cooled, filtered, and the filtrate was concentrated under reduced pressure until no fraction appeared. 90g of ethanol was added, and 90g of purified water was added dropwise. The solid was separated out under stirring, filtered, and the filter cake was vacuum dried at 47□ to obtain 16.5g of crude Efinaconazole as an off-white solid with a yield of 79.2% and a HPLC purity of 99.52%.

### Example 16

Into a reaction flask, 30g of acetonitrile, 4-methylenepiperidine hydrochloride (8.0g, 59.71mmol) and NaOH (2.4g, 59.71mmol) were added, then LiBr (13.8g, 95.5mmol) and (2R,3S)-2-(2,4-difluorophenyl)-3-methyl-[(1H-1,2,4-triazol-1-yl)methyl]oxirane (15g, 59.71 mmol) were added. The heating was started and the temperature was raised to around 85-90□, and the mixture was stirred for 14h, the heating was stopped, cooled, filtered, and the filtrate was concentrated under reduced pressure until no fraction appeared. 90g of ethanol was added, and 90g of purified water was added dropwise. The solid was separated out under stirring, filtered, and the filter cake was vacuum dried at 47□ to obtain 16.1g of crude Efinaconazole as an off-white solid with a yield of 77.3% and a HPLC purity of 99.74%.

### Example 17

Into a reaction flask, 30g of acetonitrile, 4-methylenepiperidine hydrochloride (8.0g, 59.71mmol) and NaOH (2.4g, 59.71mmol) were added, then LiBr (13.8g, 95.5mmol) and (2R,3S)-2-(2,4-difluorophenyl)-3-methyl-[(1H-1,2,4-triazol-1-yl)methyl]oxirane (15g, 59.71 mmol) were added. The heating was started and the temperature was raised to around 85-90□, and the mixture was stirred for 20h, the heating was stopped, cooled, filtered, and the filtrate was concentrated under reduced pressure until no fraction appeared. 60g of ethanol was added, and 90g of purified water was added dropwise. The solid was separated out under stirring, filtered, and the filter cake was vacuum dried at 47□ to obtain 17.5g of crude Efinaconazole as an off-white solid with a yield of 84.3% and a HPLC purity of 98.44%.

In the crystallization process of Efinaconazole, the crystallization solvents for Efinaconazole were investigated, and n-heptane, isopropyl ether, acetonitrile/water, acetone/water, ethanol/water, ethanol/acetonitrile/water, diethyl ether/n-hexane and cyclohexane were investigated respectively. The specific crystallization processes are given in the following examples.

### Example 18

25mL of n-heptane was added into 5.0g of Efinaconazole and the Efinaconazole was dissolved until clear under stirring and heating. The solution was stirred for 30min with the temperature maintained at 50-55□. The temperature was slowly lowered to 0-10□ within 2h to crystallize. The mixture was then stirred for 2h with the temperature maintained, filtered. The filter cake was vacuum dried at 47□ to obtain 4.42g of Efinaconazole as a white solid with a yield of 88.4% and a HPLC purity of 99.71%.

### Example 19

12.5mL of isopropyl ether was added into 5.0g of Efinaconazole and the Efinaconazole was dissolved until clear under stirring and heating. The solution was stirred for 30min with the temperature maintained at 50-55□. The temperature was slowly lowered to 0-10□ within 2h to crystallize. The mixture was then stirred for 2h with the temperature maintained, filtered. The filter cake was vacuum dried at 47□ to obtain 3.24g of Efinaconazole as a white solid with a yield of 64.8% and a HPLC purity of 99.55%.

### Example 20

15mL of acetonitrile was added into 5.0g of Efinaconazole and the Efinaconazole was dissolved until clear under stirring. 30mL of purified water was added dropwise, the solid was gradually separated out and the temperature was lowered to 0-10□. The mixture was then stirred for 2h with the temperature maintained, filtered. The filter cake was vacuum dried at 47□ to obtain 4.41g of Efinaconazole as a white solid with a yield of 88.2% and a HPLC purity of 99.78%.

### Example 21

15mL of acetone was added into 5.0g of Efinaconazole and the Efinaconazole was dissolved until clear under stirring. 30mL of purified water was added dropwise, the solid was gradually separated out and the temperature was lowered to 0-10□. The mixture was then stirred for 2h with the temperature maintained, filtered. The filter cake was vacuum dried at 47□ to obtain 4.74g of Efinaconazole as a white solid with a yield of 94.8% and a HPLC purity of 99.77%.

### Example 22

45mL of ethanol was added into 15.0g of Efinaconazole and the Efinaconazole was dissolved until clear under stirring. 90mL of purified water was added dropwise, the solid was gradually separated out and the temperature was lowered to 0-10□. The mixture was then stirred for 2h with the temperature maintained, filtered. The filter cake was vacuum dried at 47□ to obtain 14.35g of Efinaconazole as a white solid with a yield of 95.67% and a HPLC purity of 99.75%.

### Example 23

15mL of acetonitrile and 15mL of ethanol were added into 5.0g of Efinaconazole and the Efinaconazole was dissolved until clear under stirring. 60mL of purified water was added dropwise, the solid was gradually separated out and the temperature was lowered to 0-10□. The mixture was stirred for 12h with the temperature maintained, filtered. The filter cake was vacuum dried at 47□ to obtain 4.48g of Efinaconazole as a white solid with a yield of 89.6% and a HPLC purity of 99.74%.

### Example 24

7.5mL of diethyl ether was added into 5.0g of Efinaconazole and the Efinaconazole was dissolved until clear under stirring. 46mL of n-hexane was added dropwise, the temperature was lowered to 0-10□. The mixture was then stirred for 2h with the temperature maintained, filtered. The filter cake was vacuum dried at 47□ to obtain 3.43g of Efinaconazole as a white solid with a yield of 68.6% and a HPLC purity of 99.77%.

### Example 25

300mL of cyclohexane was added into 100g of Efinaconazole and the Efinaconazole was dissolved until clear under stirring and heating. The solution was stirred for 30min with the temperature maintained at 40-50□. The temperature was slowly lowered to 5-8□ within 2h to crystallize. The mixture was stirred for 4h with the temperature maintained, filtered, and the filter cake was vacuum dried at 47□ to obtain 81.92g of Efinaconazole as a white solid with a yield of 81.92% and a HPLC purity of 99.94%.

### Example 26

50mL of ethanol was added into 10.0g of Efinaconazole and the Efinaconazole was dissolved until clear under stirring. 60mL of purified water was added dropwise, the solid was gradually separated out and the temperature was lowered to 0-10□. The mixture was then stirred for 2h with the temperature maintained, filtered. The filter cake was vacuum dried at 47□ to obtain 8.32g of Efinaconazole as a white solid with a yield of 83.2% and a HPLC purity of 99.82%.

### Example 27

60mL of ethanol was added into 10.0g of Efinaconazole and the Efinaconazole was dissolved until clear under stirring. 60mL of purified water was added dropwise, the solid was gradually separated out and the temperature was lowered to 0-10□. The mixture was then stirred for 2h with the temperature maintained, filtered. The filter cake was vacuum dried at 47□ to obtain 7.84g of Efinaconazole as a white solid with a yield of 78.4% and a HPLC purity of 99.85%.

According to the above Examples 18-27, the purification process by cyclohexane can make the total impurities of the product less than 0.10%, the HPLC purity greater than 99.90%, and the purification yield above 80%. The purification processes by other solvents can only control the total impurities of the product to less than 0.5%. The purification yield by the ethanol/water system is between 75% and 97%, and the HPLC purity is between 99.5 and 99.9%.

### Example 28

500mL of isopropanol was added into 100g of Efinaconazole, 54.52g of p-toluenesulfonic acid was added under stirring, the temperature was raised to 70°C to dissolve the Efinaconazole and p-toluenesulfonic acid until clear, and then the temperature was lowered to 5-8°C within 2h. The mixture was stirred for 1h with the temperature maintained, filtered. The filter cake was vacuum dried at 47°C to obtain 138.5g of Efinaconazole p-toluenesulfonate as a white solid with a yield of 93.10%.

### Example 29

500mL of ethanol was added into 100g of Efinaconazole, the Efinaconazole was dissolved until clear under stirring. 54.52g of p-toluenesulfonic acid was added under stirring, the temperature was raised to 78°C to dissolve the p-toluenesulfonic acid until clear, and then the temperature was lowered to 5-8°C within 2h. The mixture was stirred for 1h with the temperature maintained, filtered. The filter cake was vacuum dried at 47°C to obtain 132.5g of Efinaconazole p-toluenesulfonate as a white solid with a yield of 89.20%.

### Example 30

50mL of ethanol was added into 10g of Efinaconazole, the Efinaconazole was dissolved until clear under stirring. 2.75g of methanesulfonic acid was added, the temperature was raised to 78°C to dissolve the methanesulfonic acid until clear, and then the temperature was lowered to 5-8°C within 2h. The mixture was stirred for 1h with the temperature maintained, filtered. The filter cake was vacuum dried at 47°C to obtain 9.96g of Efinaconazole methanesulfonate as a white solid with a yield of 78.1%.

## Claims

1. A preparation method for Efinaconazole, comprising the following steps:
in the presence of a bromide and a base, subjecting a compound of formula A and a compound of formula B to a ring-opening addition reaction in a reaction solvent to obtain a compound of formula I 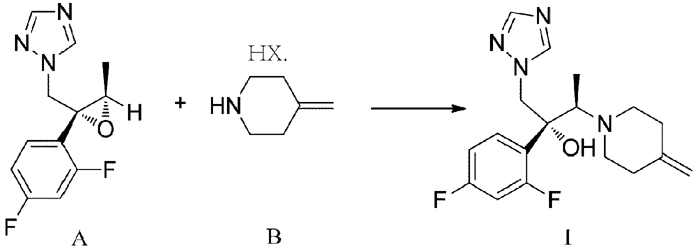 wherein, the X in the compound of formula B is Cl, Br or I, preferably Cl.

2. The method according to claim 1, wherein the base used in the reaction is one or more selected from potassium hydroxide, sodium hydroxide, potassium carbonate and lithium carbonate, preferably potassium hydroxide.

3. The method according to claim 1 or 2, wherein the reaction solvent used in the reaction is selected from acetonitrile, DMF, DME or DMSO, preferably acetonitrile.

4. The method according to any one of claims 1-3, wherein the bromide used in the reaction is lithium bromide or magnesium bromide, preferably lithium bromide.

5. The method according to any one of claims 1-4, wherein the molar ratio of the bromide to the compound of formula A is 1.5-2.5:1, preferably 2.0:1.

6. The method according to any one of claims 1-5, wherein the mass ratio of the reaction solvent to the compound of formula A is 2-5:1, preferably 2:1.

7. The method according to any one of claims 1-6, wherein the molar ratio of the base to the compound of formula A is 1.0-1.8:1, preferably 1.3:1.

8. The method according to any one of claims 1-7, wherein the molar ratio of the compound of formula A to the compound of formula B is 1:1.0-1:1.8, preferably 1:1.3.

9. The method according to any one of claims 1-8, wherein the temperature of the ring-opening addition reaction is 70-90°C, preferably 85°C; the time of the ring-opening addition reaction is 14-24h, preferably 20h.

10. The method according to any one of claims 1-9, further comprising: after completion of the reaction, subjecting a post-treatment to the product to obtain a crude Efinaconazole, wherein the post-treatment does not comprise a crystallization step.

11. The method according to claim 10, wherein the post-treatment is adding ethanol and water to the product, and wherein the mass ratio of ethanol to water is 5:6-6:6, preferably 5.5:6.

12. The method according to any one of claims 1-11, further comprising a crystallization step.

13. The method according to claim 12, wherein the crystallization step comprises: adding the crude Efinaconazole to a crystallization solvent, stirring and crystallizing, and filtering to obtain pure Efinaconazole.

14. The method according to claim 13, wherein, in the crystallization step, the crystallization temperature is 0-25°C, preferably 0-10°C, more preferably 5-8°C; the time for stirring and crystallizing is 2-15 hours, preferably 4-14 hours.

15. The method according to claim 12, wherein the crystallization solvent used in the crystallization step is selected from n-heptane, isopropyl ether, acetonitrile/water, acetone/water, ethanol/water, ethanol/acetonitrile/water, diethyl ether/n-hexane or cyclohexane, preferably cyclohexane.

16. The method according to any one of claims 1-15, wherein the obtained compound of formula I is formed into a salt with an acid.
